# EUROPEAN PATENT SPECIFICATION

(11) **EP 1 368 496 B1**
(45) Date of publication and mention of the grant of the patent: **22.09.2010**
(21) Application number: 02725179.2
(22) Date of filing: 15.03.2002
(51) Int. Cl.: C12Q 1/68

(54) **Method for alteration detection in nucleic acids**
Verfahren zum Nachweis einer Mutation in einer Nukleinsäure
Méthode de détection d'une alteration d'un acide nucléique

(30) Priority: 15.03.2001 US 809713; 20.11.2001 US 988491
(43) Date of publication of application: 10.12.2003
(73) Proprietor: Genzyme Corporation, Cambridge, MA 02142 (US)
(72) Inventor: SHUBER, Anthony, P., Mendon, MA 01756 (US)
(74) Representative: Crump, Julian Richard John
(86) International application number: PCT/US2002/007926
(87) International publication number: WO 2002/074995

(56) References cited:
- WO-A-01/27326
- WO-A-99/19517
- US-A- 6 025 127
- US-A- 6 051 359
- US-A- 6 051 378
- SHENK T E ET AL: "Biochemical method for mapping mutational alterations in DNA with S1 nuclease: the location of deletions and temperature-sensitive mutations in simian virus 40." PROCEEDINGS OF THE NATIONAL ACADEMY OF SCIENCES OF THE UNITED STATES OF AMERICA MAR 1975, vol. 72, no. 3, March 1975 (1975-03), pages 989-993, XP002442559 ISSN: 0027-8424
- BROOKES A J ET AL: "Evaluation of the use of S1 nuclease to detect small length variations in genomic DNA." EUROPEAN JOURNAL OF BIOCHEMISTRY / FEBS 1 AUG 1989, vol. 183, no. 2, 1 August 1989 (1989-08-01), pages 291-296, XP002442560 ISSN: 0014-2956
- YE SHENG ET AL: "Detection of single nucleotide polymorphisms by the combination of nuclease S1 and PNA." NUCLEIC ACIDS RESEARCH. SUPPLEMENT (2001) 2002, no. 2, 2002, pages 235-236, XP002442561
- SEITZ O ET AL: "Convergent strategies for the attachment of fluorescing reporter group" CHEMISTRY - A EUROPEAN JOURNAL, VCH PUBLISHERS, US, vol. 7, no. 18, 17 September 2001 (2001-09-17), pages 3911-3925, XP002408947 ISSN: 0947-6539
- MURRAY M G: "USE OF SODIUM TRICHLOROACETATE AND MUNG BEAN NUCLEASE TO INCREASE SENSITIVITY AND PRECISION DURING TRANSCRIPT MAPPING" ANALYTICAL BIOCHEMISTRY, vol. 158, no. 1, 1986, pages 165-170, XP009093382 ISSN: 0003-2697
- STILGENBAUER S ET AL: "Molecular characterization of 11q deletions points to a pathogenic role of the ATM gene in mantle cell lymphoma" BLOOD, vol. 94, no. 9, 1 November 1999 (1999-11-01), pages 3262-3264,
- EGGERDING F A: "FLUORESCENT OLIGONUCLEOTIDE LIGATION TECHNOLOGY FOR IDENTIFICATION OF RAS ONCOGENE MUTATIONS" MOLECULAR BIOTECHNOLOGY, vol. 14, no. 3, 1 March 2000 (2000-03-01), pages 223-233, XP008040192

### SEQUENCE LISTING

<110> Exact Sciences Corporation Shuber, Anthony
<120> Method For Alteration Detection
<130> EXT-047PC
<150> US 09/809,713
   <151> 2001-03-15
<150> US 09/988,491
   <151> 2001-11-20
<160> 5
<170> PatentIn version 3.0
<210> 1
   <211> 51
   <212> DNA
   <213> Homo sapiens
<400> 1
   ctccaccacc tcctcaaaca gctcaaacca agcgagaagt acctaaaaat a 51
<210> 2
   <211> 17
   <212> DNA
   <213> Artificial
<220>
<223> Probe designed for the region of codon 1450
<400> 2
   cgcttggttt gagctgt 17
<210> 3
   <211> 17
   <212> DNA
   <213> Artificial
<220>
   <223> probe upstream of the 1450 point mutation region
<400> 3
   ttgaggaggt ggtggag 17
<210> 4
   <211> 17
   <212> DNA
   <213> Artificial
<220>
   <223> probe downstream of the 1450 point mutation
<400> 4
   tatttttagg tacttct 17
<210> 5
   <211> 17
   <212> DNA
   <213> Homo sapiens
<400> 5
   acagctcaaa ccaagtg 17

## Description

### FIELD OF THE INVENTION

The invention relates generally to methods for detecting an alteration in a target nucleic acid.

### BACKGROUND OF THE INVENTION

Many diseases are associated with genomic instability. As such, instability markers have been proposed as diagnostics. For example, mutations are considered valuable markers for a variety of diseases, and have formed the basis for screening assays. Specific mutations might be a basis for molecular screening assays for the early stages of certain types of cancer. *See, e*.*g.,* Sidransky, et al., Science, 256: 102-105 (1992*).* For example, mutations in the BRCA genes have been proposed as markers for breast cancer, and mutations in the p53 cell cycle regulator gene have been associated with the development of numerous types of cancers.

Early alteration detection allows early disease diagnosis, and thus also provides an avenue for intervention prior to the presentation of disease symptoms that often occurs after metastasis when a cure is less readily attainable. However, the detection of genetic mutations or other alterations is difficult, or impossible, in certain sample types. For example, the difficulty of isolating DNA from complex, heterogeneous samples makes identification of early-stage mutation difficult.

Therefore, there is a need in the art for efficient methods for determining the presence or absence of certain genetic mutations or other alterations in a target nucleic acid in a biological sample.

US 6,051,378 relates to methods for screening nucleic acids for mutations by analyzing nonrandomly fragemented nucleic acids using mass spectrometric techniques and to procedures for improving mass resolution and mass accuracy of these methods.

Shenk et al. Proc. Nat. Acad. Sci. USA 1975, vol. 72, no. 3, March 1975, pages 989-993 relates to a biochemical method for mapping mutational alterations in DNA with S1 nuclease.

Seitz et al. Chem. Eur. J. US, vol. 7, no. 18, 17 September 2001 (2001-09-17), pages 3911-3925 relates to strategies for the attachment of fluorescing reporter groups to peptide nucleic acids in solution and on solid phase.

Brookes et al. (1989) Eur. J. Biochem., vol. 183, no. 2 relates to the evaluation of the use of S1 nuclease to detect small length variations in genomic DNA.

WO99/19517 relates to the analysis of nicked DNA by matched ion polynucleotide chromatography.

### SUMMARY OF THE INVENTION

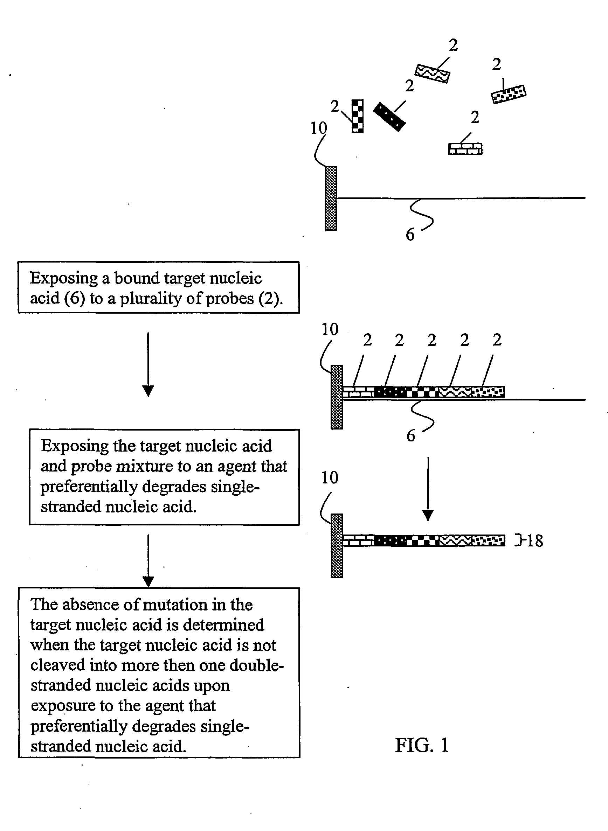

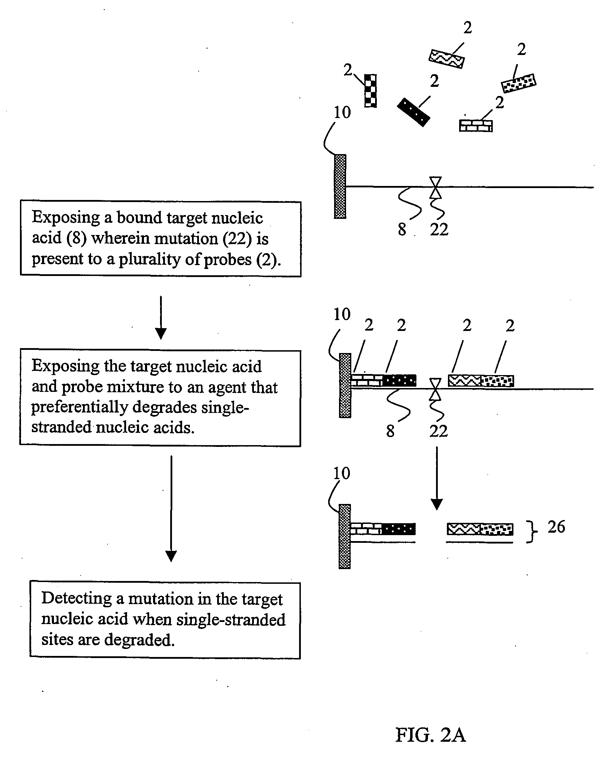

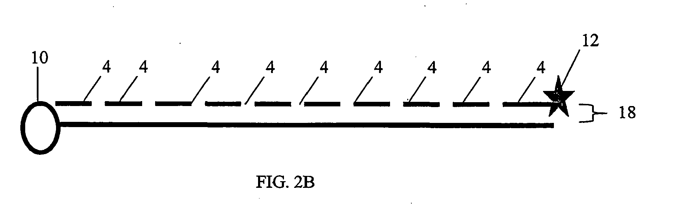

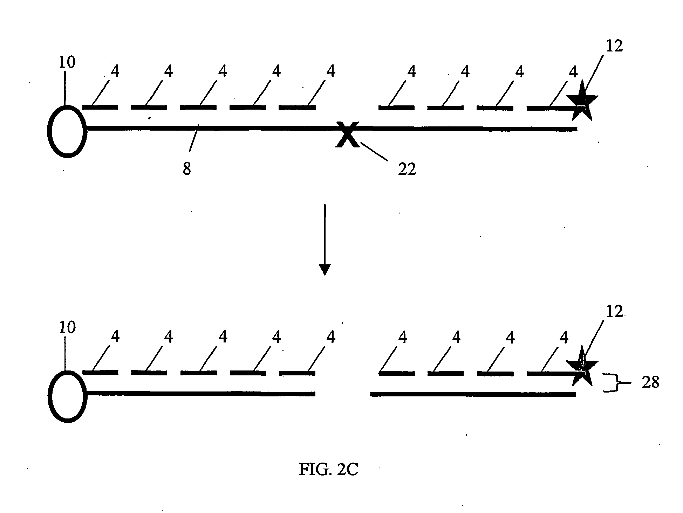

The invention provides methods for detecting an alteration in a target nucleic acid in a biological sample. According to the invention, a plurality of single stranded nucleic acid or peptide nucleic acid probes complementary to a contiguous region of target nucleic acid if said region is unaltered are exposed to biological sample suspected to contain the target. The method further comprises adding to said biological sample an agent that degrades single-stranded nucleic acids; and detecting an alteration in said target nucleic acid as the presence of a degredation product resulting from degredation within said region of said target nucleic acid. Probes are designed to hybridize to the target in a contiguous manner to form a duplex comprising the target and the contiguous probes"tiled"along the target. An example of this duplex is shown in Figure 1. If a mutation or other alteration exists in the target, contiguous tiling will be interrupted, producing regions of single-stranded target in which no duplex exists. This is shown in Figure 2. Identification of one or more single-stranded regions in the target is indicative of a mutation or other alteration in the target that prevented probe hybridization in that region. For purposes of the present invention, a"tiled sequence" or"tiling"refers to the contiguous hybridization of probes to a target region, whether separated by single-stranded sequence or not.

Accordingly, in methods of the invention, a sample comprising a singlestranded target nucleic acid is exposed to a plurality of nucleic acid probes. In a preferred embodiment, the plurality of probes comprises probes that are complementary to different positions of the target such that hybridization of members of the plurality with a wild-type target results in a contiguous series of probes along at least a portion of the target sequence when the target is a wild-type target. Probes including RNA, DNA and/or Peptide Nucleic Acid (PNA) may be employed to hybridize to the target nucleic acid. It is not necessary to ligate the series of probes to form a continuous strand, although ligation may be performed at the discretion of the user.

When the target is a wild-type sequence, there will be no single-stranded portion in the region in which the probes are tiled. However, when a mutation or other alteration exists in the region of the target to which probes are directed, one or more of the probes will fail to hybridize, resulting in one or more single-stranded portions of the target region. Identification of this single-stranded region is, according to the invention, a positive assay for a mutation or other alteration in the target.

In a preferred embodiment, a single-stranded region indicative of a mutation in the target is detected by exposing the target, subsequent to probe hybridization, to an agent that selectively cleaves single-stranded nucleic acid. In a mutated target, methods of the invention produce more than one "tiled" duplex in the target region. Multiple double-stranded tiled duplexes result from cleavage of the target in the single-stranded region to which any probe failed to hybridize. Numerous cleavage enzymes are known which selectively cleave or degrade single-stranded nucleic acids (*e*.*g*., S1, MutY, MutS and Mungbean nuclease). Identification of a single contiguous duplex comprising the target and the contiguous tiled probes upon exposure to the selective cleavage or degradation agent is indicative of a wild-type (non-mutated) target region. Alternatively, the products of cleavage are measured to determine, for example, whether the molecular weight of the products is different than would be expected from a single contiguous duplex.

According to the invention, single-stranded nucleic acids can be generated to form the target by standard methods known in the art. Such methods include denaturing double stranded nucleic acids and/or generating single stranded nucleic acids in an *in*-*vitro* enzyme catalyzed reaction wherein an excess of one strand is generated. Preferred methods include capturing a single stranded target nucleic acid on a solid or semi-solid support.

Also in a preferred embodiment, the assay described above is multiplexed in order to interrogate multiple targets simultaneously. As such, one can look for specific double-stranded cleavage products in order to identify the specific mutated target(s) or one can simply identify multiple cleavage products (resulting, as described above, from intervening single-stranded regions in the "tiled target") as evidence of a mutation at one of the interrogated targets. For example, multiple targets, each containing a so-called "hot spot" for mutation in cancer are interrogated, the production of a single-stranded target region after tiling being sufficient to result in a positive screen for cancer or pre-cancer.

Also, in yet another preferred embodiment, a heterogeneous sample is diluted by adding buffer, for example, sample or assay buffer, and the diluted sample is then separated into fractions. Preferred fractions contain on average 1-5 nucleic acid molecules from the original heterogeneous sample. The separate sample fractions are preferably amplified by, for example, PCR, which concentrates each separate sample fraction of target nucleic acid. As a result, each separate fraction is enriched for a subset of nucleic acid molecules that were present in the original heterogeneous sample. Accordingly, when probes complementary to the wild-type sequence are added to each separate sample fraction, a mutation that is enriched in one of the separate fractions may be detected as an enhanced signal. Such methods may be employed, for example, to detect rare events in the sample.

Methods of the invention are also useful for detecting non-hybridized regions at the termini of a target. When a mutation occurs in a region of target to which a terminal tile would hybridize if the target is a wild-type target, the resulting degradation of the single-stranded terminus will not, as described above, produce multiple duplex products indicative of an intervening single-stranded region. Instead, the terminal single-stranded region will be cleaved or degraded, leaving the tiled portion of the target intact. In that case, the terminal mutation is identified in by the reduced expected molecular weight of the tiled target or by the activity of the degrading agent (e.g., an exonuclease).

Alternatively, a mutation or other alteration in the termini of a target may also be detected by evaluating both the sense strand and antisense strand of the target. According to methods of the invention, both the sense and antisense strands of the target are bound to a solid support by the same respective terminus; for example, both the sense and the antisense strands of the target are bound to a solid support by their respective 5' ends. Thereafter, the bound sense and antisense strands of the target are interrogated in solution. A terminal mutation on, for example, the unbound 3' end of the sense strand would go undetected, however, the mutation presents a duplex cleaved from the mutation site near the bound 5' end of the antisense strand. The mutation is detected when the solid support is removed and the duplex cleaved off of the antisense strand remains in solution. If only the sense strand were tested, then the mutation would go undetected, thus testing both the sense and the antisense strands avoids a false negative caused by a terminal mutation on one of the strands.

Methods of the invention may be used to avoid detecting a known polymorphism as a false positive for an alteration. One or more polymorphisms may be present in a region of the target nucleic acid. In that case, multiple probes are designed to hybridize to each of the polymorphic variants known to be present in the target region. The probe complimentary to the polymorphism variant that is present in the target will hybridize to the region of the polymorphism. Providing the range of variants complementary to the associated polymorphisms ensures that the polymorphism region is "tiled" and any positive signal detected indicates the presence of an alteration other than the associated polymorphism on the target nucleic acid.

Methods of the invention are also useful for detecting the presence, in a population, of one or more polymophisms on a target nucleic acid. A plurality of nucleic acid probes is complementary to a first variant of a target nucleic acid comprising a polymorphism such that hybridization of members of the plurality with the target comprising the polymorphism variant results in a contiguous series of probes along at least a portion of the target. When one or more other polymorphic variants are present in the region of the target to which the probes are directed, at least one of the probes will fail to hybridize at the site of the other polymorphic variant. After hybridization, a single-stranded portion of the target region is exposed at the site of, for example, a second polymorphic variant. Upon exposure to an agent that selectively degrades single-stranded nucleic acid, the polymorphic variant presents a duplex cleaved from, for example, the site of the second polymorphic variant. Accordingly, detection of a cleavage product is a positive assay for an alteration, in this case the one or more other polymorphic variants, in the target.

In another aspect, probes are designed to hybridize to the target such that gaps separate one or more of the hybridized probes from each other. According to this aspect of the invention, a sample comprising single-stranded target nucleic acid is exposed to a plurality of nucleic acid probes. The plurality comprises probes that are complementary to different positions on the target nucleic acid such that hybridization of members of the plurality with a wild-type target results in a series of probes, along at least a portion of the target sequence, some of which may be separated a gap.

Preferably, the gaps are small enough to prevent the degradation agent from cutting at the gap. Accordingly, preferred gap separations are shorter than the probes, i.e., the probes have a higher number of base pairs then the gaps. In preferred embodiments, the gap separations range between about 1 base pair and about 3 base pairs in length. Alternatively, gaps can be between about 3 base pairs and 15 base pairs in length. According to the invention, a detection assay can be performed using a plurality of probes some of which are separated by gaps and some of which are not when hybridized to the target nucleic acid.

According to this aspect of the invention, the degradation agent that is selected, or alternatively, the conditions employed with the agent, do not cleave the target nucleic acid at the single-stranded gaps. In some embodiments, milder degradation conditions that avoid degrading any single-stranded gap separations are employed. For example, the number of degradation agent units used are held at a temperature and for a time period appropriate to maintain the single-stranded gap separations between tiles. The degradation agent conditions simultaneously degrade larger single-stranded regions where one or more probes failed to hybridize due to an alteration in the target. Because the gap separations are not degraded, false positives created by gap separations between the probes are avoided. However, the conditions enable degradation of the target region where the probe failed to hybridize, avoiding a false negative. Thus, according to the invention, a positive assay for an alteration in a target nucleic acid may comprise gaps separating complementary probes hybridized to the target. Where gap separations are present between probes, the selected degradation agent, units of agent used, temperature and exposure time are selected to provide conditions that maintain single stranded gap separations and that simultaneously cleave a single stranded alteration region where a probe failed to hybridize. Steric hindrance can also be used to prevent cutting at the gaps.

The probes may be designed such that they prevent adjacent hybridized probes from creating a stabilizing effect that results in a probe hybridizing at the site of an alteration in a region of the target nucleic acid. Accordingly, the probes themselves are altered to include between about 1 and about 10 Abasic sites on the 5' and/or the 3' end of one or more of the probes. Similarly, the probes may be altered to put between about 1 to about 10 DNA, RNA, or Peptide Nucleic Acids that do not match the target nucleic acid on the 5' and/or the 3' end, and that do not prevent the probe from hybridizing to its complementary target.

In a preferred embodiment, a target nucleic acid is bound to a solid-support at either its 3' or 5' terminus. Complementary probes are tiled along the length of the target as described above. A mutation is indicated when double-stranded hybridization products are detected in solution after the sample is treated with a degradation agent indicating that one or more tiling probes failed to hybridize to the target due to the mutation. More than one target nucleic acid from more than one source can be simultaneously screened by binding multiple target nucleic acids to solid supports. Also, double-stranded nucleic acid can be melted by, for example, heating.

In the event that a mutation is detected on a target nucleic acid, the identity of the mutation is determined by any method known in the art, such as sequencing, mass spectroscopy, and others.

In a preferred embodiment, a biological sample is exposed to probes complementary to a target DNA under stringent hybridization conditions so that each probe will hybridize only to the wild-type target nucleic acid. Such conditions are well-known in the art. *See*, *e*.*g*., 2 Joseph Sambrook, Peter MacCallum, & David Russell, Molecular Cloning: A Laboratory Manual ch., 10 (3d ed. 2001).
In one embodiment, the hybridization melting temperature of each probe is about the same. In another embodiment, the probes are between about 8 and about 30 nucleotides long. In one preferred embodiment, each probe is the same length i. e. composed of the same number of nucleotides.

Preferred biological samples are sputum, pancreatic fluid, bile, lymph, plasma, urine, cerebrospinal fluid, seminal fluid, saliva, breast nipple aspirate, pus, biopsy tissue, fetal cells, amniotic fluid, and stool.

In another embodiment, at least one of the tiling probes comprises a detectable label. Each probe may comprise a different detectable label, permitting the differential detection of the probes (i.e., for example, the different probes may comprise a nucleotide with a different radioactive isotope, a fluorescent tag, or a molecular weight modifying entity). Differential probe labeling allows the identification of the probe that did not anneal to its target in the case of a mutation. Each probe or a subset of probes may be labeled and/or all or a portion of the target nucleic acid may be labeled. In a preferred embodiment, all probes are labeled. In a more preferred embodiment the target nucleic acid is bound to a solid support, and a label, disposed on either the target, the probes, or both, is distal preferably most distal, i.e., at the unbound end, to the position of an alteration.

In another embodiment, the target nucleic acid comprises a detectable label in the region at which a mutation is suspected. When the suspected mutation is present in the target, no probe will hybridize to the target and the region of the mutation comprising the detectable label will remain single stranded. Upon exposure to an agent that cleaves single-stranded nucleic acid, the single-stranded mutation region comprising the detectable label is degraded from the target. The absence of the label in the degradation products is indicative of the presence of a mutation in the region of the detectable label.

The means of alteration identification, including, for example, probe labeling, probe preparation, for example, to a solid support, the disease associated mutations, sample sources, degradation agents, and other embodiments and illustrative examples may apply to all of the alteration detection methods disclosed herein. For example, fluorescently labeled probes may be hybridized to the target in a contiguous manner, the fluorescently labeled probes may be separated by gaps, or the fluorescently labeled probes may complimentary to polymorphic variants present in the target. In another embodiment, a target that is bound to a solid support may be exposed to probes: that hybridize to the target in a contiguous manner, that hybridize to the target with gap separations, or that hybridize to polymorphic variants present in the target.

In preferred embodiments of the invention, different patient samples, different fractions of a patient sample, or a combination thereof, are analyzed simultaneously, for example, in a two-dimensional array such as on a multiwell plate, using methods of the invention.

In one embodiment, methods of the invention comprise detecting a mutation at a genetic locus that is associated with a disease, such as K-RAS, p53, APC, DCC, or BAT26. In a preferred embodiment, that mutation is associated with cancer, such as colon cancer, lung cancer, esophageal cancer, prostate cancer, breast cancer, pancreatic cancer, stomach cancer, liver cancer, or lymphoma.

A detailed description of certain embodiments of the invention is provided below. Further aspects and advantages of the invention are apparent upon consideration of the following drawings, description and claims.

### DESCRIPTION OF THE DRAWINGS

Figure 1 shows a flow chart diagram that illustrates an embodiment of a method of the invention of detecting the absence of mutation in a target nucleic acid sample.

Figure 2A shows a flow chart diagram that illustrates an embodiment of a method of the invention for detecting the presence of mutation in a target nucleic acid sample.

Figure 2B illustrates another embodiment of the method of the invention for detecting the absence of mutation in a target nucleic acid sample.

Figure 2C shows a flow chart diagram that illustrates another embodiment of a method of the invention for detecting the presence of mutation in a target nucleic acid sample.

Figure 3 shows results from a gel where polymorphic variant probes are selectively employed to block polymorphic variants of the target nucleic acid sample.

Figure 4A illustrates a probe having a reporting moiety and a quenching moiety.

Figure 4B illustrates the reporting moiety of a first probe of Figure 4A annealed to the quenching moiety of a second probe.

Figure 4C shows a flow chart diagram that illustrates another embodiment of a method of the invention for detecting the absence of mutation in a target nucleic acid sample.

Figure 5 shows a flow chart diagram that illustrates another embodiment of a method of the invention for detecting the presence of mutation in a target nucleic acid sample.

### DETAILED DESCRIPTION OF THE INVENTION

The present invention provides methods for detecting a genetic alteration in target nucleic acids indicative of genomic instability. For example, methods of the present invention are useful to detect and/or to identify mutations or other alterations associated with diseases, such as cancer and other pathological genetic conditions, disorders or syndromes. Such mutations include nucleotide insertions, deletions, rearrangements, transitions, translations, tranversions, polymorphisms, and substitutions. The present invention may be used to identify inherited mutations or other alterations, such as induced or spontaneous sporadic mutations. Generally, however, alterations include any change in the target nucleic acid, such as a mutation, loss of heterozygosity, or other indicia of genomic instability.

Methods of the invention rely upon the use of a plurality of probes, each probe comprises single-stranded nucleic acids and each probe is complementary to a different portion of a contiguous region of the target nucleic acid. According to the invention, each probe hybridizes to its complementary region on the target nucleic acid. When no mutation or other alteration is present in the target, the plurality of probes form a contiguous "tile" along the length of the target region. In the event that a portion of the target contains a mutation or other alteration, the target remains single-stranded in that region because the otherwise complementary probe will fail to hybridize in the presence of the mutation. Identification of the single-stranded region is indicative of a mutation or other alteration.

In a preferred embodiment, a single-stranded region indicative of a mutation or other alteration is detected by exposing the tiled target to an agent that preferentially degrades or cleaves single-stranded nucleic acid, and analyzing the degradation product(s). Exemplary degradation agents include chemical agents and enzymes, such as S1, MutY, MutS, and Mungbean nuclease. The presence of a singular intact double-stranded nucleic acid product is indicative of the absence of a mutation in any of the regions of the target nucleic acid (i.e., no cleavage of the target due to the absence of a single-stranded portion). The presence of two or more double-stranded products is indicative of the presence of a mutation or other alteration in one or more of the regions of the target nucleic acid (evidencing cleavage of the target at the single-stranded region(s) containing the mutation).

Biological samples that are useful in the present invention include any sample from a patient in which a target nucleic acid is present. Such samples are prepared from any tissue, cell, or body fluid. Examples of biological cell sources include blood cells, colon cells, buccal cells, cervicovaginal cells, epithelial cells from urine, fetal cells or cells present in tissue obtained by biopsy. Exemplary tissues or body fluids include sputum, pancreatic fluid, bile, lymph, plasma, urine, cerebrospinal fluid, seminal fluid, saliva, breast nipple aspirate, pus, amniotic fluid and stool. Useful biological samples also include isolated nucleic acid from a patient. Nucleic acid can be isolated from any tissue, cell, or body fluid using any of numerous methods that are standard in the art. The particular nucleic acid isolation method will depend on the source of the patient sample.

The biological sample comprising a target nucleic acid may be analyzed by methods of the present invention without further preparation or purification. In a preferred embodiment, one or more specific regions present in the target nucleic acid may be amplified by, for example, PCR. Concentrating the target nucleic acid by amplification improves accuracy by reducing background noise in the sample.

In one embodiment, the target nucleic acid is bound to a solid phase or semi-solid phase matrix. Support binding allows the simultaneous processing and screening of a plurality of nucleic acid samples from different sources, and allows degradation products to be compared in the liquid phase. Exemplary matrices suitable for use in the present invention include nitrocellulose or nylon filters, glass beads, magnetic beads coated with agents for affinity capture, treated or untreated microtiter plates, polymer gels, agarose and the like. It will be understood by a skilled practitioner that the method by which the target nucleic acid is bound to the matrix will depend on the particular matrix used. For example, binding to nitrocellulose can be achieved by simple absorption of nucleic acid to the filter followed by baking the filter at 75°-80° C under vacuum for 25 minutes to 2 hours. Alternatively, charged nylon membranes that do not require any further treatment of the bound nucleic acid can be used. Beads and microtiter plates that are coated with avidin can be used to bind target nucleic acid to which biotin is attached (by, for example, the use of biotin-conjugated PCR primers). In addition, antibodies can be used to attach target nucleic acid to any of the above solid supports by coating the surfaces with an antibody and incorporating an antibody-specific hapten into the target nucleic acid. Excess binding agents are removed from the bound target nucleic acid by washing with appropriate buffers.

In practicing the present invention, the target nucleic acid, preferably bound to a solid phase or semi-solid phase matrix, is incubated with a plurality of nucleic acid probes. The length of individual probes may be 8-100 nucleotides. In a preferred embodiment, individual probes are 8-30 nucleotides in length. In a more preferred embodiment, probes are about 17 nucleotides in length. Probes comprising RNA, DNA, and/or Peptide Nucleic Acid (PNA) may be employed to hybridize to the target nucleic acid. The probes may be synthesized chemically by methods that are standard in the art, *e*.*g*., using commercially-available automated synthesizers. One or more of the probes may be labeled. For example, fluorochromes (such as FITC or rhodamine), enzymes (such as alkaline phosphatase), biotin; or other well-known labeling compounds may be attached directly or indirectly. Alternatively, the probes may be radioactively labeled (*e.g*., end-labeled with ³²P using polynucleotide kinase) or conjugated to other commonly used labels or reporter molecules. Further, these oligonucleotides can be marked with a molecular weight modifying entity (MWME) that uniquely identifies each of the probes.

As described in Shuber et al., Human Molecular Genetics, 2:53-158, (1993*),* the hybridization reaction can be performed under conditions in which probes having different nucleic acid sequences hybridize to their complementary DNA with equivalent strength. This is achieved by: 1) employing probes of equivalent length; and 2) including in the hybridization mixture appropriate concentrations of one or more agents that eliminate the disparity in melting temperatures (Tₘ) among probes of identical length but different guanosine+cytosine (G+C) content. Thus, under these conditions, the hybridization melting temperatures (Tₘ) of each member of the plurality of single-stranded nucleic acids is approximately equivalent. Agents that may be used for this purpose include quaternary ammonium compounds such as tetramethylammonium chloride (TMAC).

TMAC reduces hydrogen-bonding energy between G-C pairs. At the same time, TMAC increases the thermal stability of hydrogen bonds between A-T pairs. Those opposing influences reduce the difference in normal bond strength between the triple-hydrogen bonded G-C based pair and the double-hydrogen bonded A-T pair. TMAC also increases the slope of the melting curve for each probe. Together, those effects allow the stringency of hybridization to be increased to the point that single-base differences can be resolved, and non-specific hybridization minimized. *See, e.g.,* Wood et al., Proc. Natl. Acad. Sci., U.S.A. 82:1585, (1985*)*.
Any agent that exhibits those properties can be employed in practicing the present invention. Such agents are easily identified by determining melting curves for different test probes in the presence and absence of increasing concentrations of the agent. This can be achieved by attaching a target nucleic acid to a solid matrix such as a nylon filter, individually hybridizing radiolabeled probes of identical lengths but different G+C content to the filter, washing the filter at increasing temperatures, and measuring the relative amount of radiolabeled probe bound to the filter at each temperature. Any agent that, when present in the hybridization and washing steps described above, results in approximately superimposable and steep melting curves for the different oligonucleotides may be used.

In practicing the present invention, the target nucleic acid and probes are incubated for sufficient time and under appropriate conditions to maximize specific hybridization and minimize non-specific hybridization. The conditions to be considered include the concentration of each probe, the temperature of hybridization, the salt concentration, and the presence or absence of unrelated nucleic acid.

The concentration of each probe generally ranges from about 0.025 to about 0.2 pmol per ml of hybridization solution. In one embodiment, each of the probes comprises an equal number of nucleotides. The probe sequences are designed to hybridize to consecutive, adjacent regions of the target nucleic acid. The optimal concentration for each probe can be determined by test hybridizations in which the signal-to-noise ratio (*i*.*e*., specific versus non-specific binding) of each probe is measured at increasing concentrations of labeled probes.

The temperature for hybridization can be optimized for the length of the probes being used. This can be determined empirically, using the melting curve determination procedure described above. It will be understood by skilled practitioners that hybridization condition determination of optimal time, temperature, probe concentration, salt type, and salt concentration should be done in concert.

According to the methods of the present invention, tiling probes hybridize only to their complementary region on the target nucleic acid. Thus, the target nucleic acid will remain single-stranded at any locus at which a mutation is present because no probe will hybridize at that locus. An exemplary alteration includes a single nucleotide polymorphism. Following hybridization, unbound probes are, if necessary, removed by washing under conditions that preserve perfectly matched target nucleic acid:probe hybridization products. Washing conditions such as temperature, time of washing, salt types and salt concentrations are determined empirically as described above.

Methods of the invention also avoid known polymorphisms being detected as a false positive for an alteration. Where one or more polymorphisms are associated with a region of the target nucleic acid, multiple probes, each designed to hybridize to one of the polymorphic variants are provided. A probe complimentary to a polymorphic variant on the target will hybridize to the region of the polymorphism. Thus, according to the method, probes are designed to block the polymorphic variants such that where the target is otherwise unaltered, the probes hybridize contiguously to a region of the target nucleic acid. Thus, providing probes complementary to each polymorphic variant ensures that the polymorphic region is "tiled" and any single-stranded regions detected according to the method indicate the presence on the target nucleic acid of an alteration other than an associated polymorphic variant.

In another aspect, probes are designed to hybridize to the target such that gaps separate one or more of the hybridized probes. According to this aspect of the invention, a sample comprising single-stranded target nucleic acid is exposed to a plurality of nucleic acid probes. The plurality comprise probes that are complementary to different positions of the target nucleic acid such that hybridization of members of the plurality with a wild-type target results in a series of probes, along at least a portion of the target sequence, that are separated by a gap of single-stranded nucleic acid. The gaps are sized such that the probes are longer then the gap separations, i.e., the probes have a higher number of base pairs then the gaps. In some embodiments, the gaps range between about 1 base pair and about 3 base pairs in length. Alternatively, gaps can range between about 3 base pairs and about 15 base pairs in length.

According to this aspect of the invention, the agent that is selected, or alternatively, the conditions employed with the agent, do not cleave the target nucleic acid at the single-stranded gap separations. In some embodiments, a milder degradation agent is employed under conditions selected to avoid degrading single-stranded gap separations, such as, for example, the agent Mungbean nuclease is exposed at a temperature of about 37°C for a period of about 10 minutes. Where gap separations are present between probes, the selected degradation agent, units of agent used, temperature and exposure time are selected to provide conditions that maintain (i.e., do not cut) single-stranded gap separations in the nucleic acid. However, the selected conditions degrade the single-stranded region of the nucleic acid where one or more probes failed to hybridize due to an alteration in the target. Because the gap separations do not degrade, false positives created by gap separations between the probes are avoided. However, the conditions enable degradation of the region of single-stranded nucleic acid on the target where the probe failed to hybridize, avoiding a false negative. Thus, according to the invention, a positive assay for an alteration in a target nucleic acid may comprise single-stranded gaps on the target separating complementary probes hybridized to the target.

In one embodiment, the target nucleic acid is present at a higher concentration than each individual probe, at least one of which is labeled with, for example, a fluorescent label that can be detected by excitation at the specific absorption wavelength from a light source in a spectrophotometer (fluorescent reporter). The hybridization products are removed from the solution, and the solution is evaluated for fluorescence. If no mutation is present in the target nucleic acid, no labeled probe should remain in the solution as all of the labeled probes will be bound to the target nucleic acid. Thus, the absence of mutation in the target nucleic acid is indicated if the solution does not fluoresce at an appreciable level. Alternatively, if the target nucleic acid is solid-support bound, the fluorescence of hybridized nucleic acid in solution after exposure to a degradation agent is indicative of the presence of a mutation in the target nucleic acid.

In another embodiment, the probe is radioactively labeled or chemiluminescent probes are employed and the presence of a mutation in the target nucleic acid is determined by exposure to X-ray film. Alternatively, or in addition, probes may carry a molecular weight modifying entity (MWME) that is unique for each probe. Such an entity allows direct identification of the separated probe by determination of the relative molecular weight by any number of methods.

While immobilization of the target nucleic acid is generally preferred, in some embodiments it may be desirable to hybridize the tiling probes to the target nucleic acid in solution. After exposing the hybridization product in solution to a degradation agent that preferentially degrades single-stranded nucleic acid, the degradation product(s) is analyzed by methods of the art that include SDS polyacrylamide gel electrophoresis, mass spectrophotometer, chromatography, hybridization capture and others. *See*, Ausubel et al., Short Protocols in Molecular Biology, 3rd ed. (John Wiley & Sons, Inc., 1995); Wu Recombinant DNA Methodology II, (Academic Press, 1995).

After detection of a mutation, the region, or genetic locus in the target nucleic acid where the mutation is present may be determined by identification of specific probes that failed to hybridize to the target nucleic acid. For example, in one embodiment, the hybridization product is cleaved into two separate double-stranded nucleic acids upon treatment with a degradation agent that preferentially degrades single-stranded nucleic acid. The two nucleic acids are separated and sequenced according to methods known in the art. The relative location and identity of the probes that successfully hybridize to the target nucleic acid can then be determined. Through the process of elimination, the one or more probes that failed to hybridize can be identified, as well as their relative position on the target nucleic acid. The genetic locus having a mutation will have a corresponding wild-type that is complementary to the probe that failed to hybridize.

Figure 1 shows a flowchart diagram illustrating an embodiment of the present invention. As shown in Figure 1, the absence of a mutation in a target nucleic acid is determined when the target nucleic acid is not cleaved into two or more double stranded fragments. In general overview the method comprises the steps of: exposing a bound target nucleic acid to a plurality of probes; exposing the target nucleic acid and probe mixture to an agent that preferentially degrades single-stranded nucleic acids; and determining that there is an absence of a mutation in the target nucleic acid if a singular intact double-stranded nucleic acid product is present in the sample after exposure to the degradation agent.

More specifically, the target nucleic acid (6) is bound to a solid phase or semi-solid phase matrix (10). The target nucleic acid is exposed to a plurality of probes (2) that are labeled with, for example, a fluorescent molecule. The target nucleic acid (6) and the plurality of probes (2) are incubated under optimal time, temperature, probe concentration, salt type, and salt concentration conditions. Stringent hybridization conditions that maximize specific hybridization by improving bonding energy symmetry and providing similar melting temperatures for each probe are employed. Those hybridization conditions enable only complementary probes to hybridize to the target nucleic acid. The target nucleic acid (6) and probe (2) mixture is then exposed to a degradation agent that preferentially degrades single-strand nucleic acid. The agent may be, for example, S1 nuclease.

The hybridization product comprising the target nucleic acid and probes (18) is then removed by its bound end from solution. The use of bound target nucleic acid enables a number of samples to be screened simultaneously by removing the bound portion from solution (for example by removing the supernatant from a reaction mixture containing bound target) then analyzing the solution phase for degradation product indicative of a mutation.

Figure 2A shows a flowchart diagram illustrating an embodiment of the present invention. In general overview, the method comprises the steps of: exposing a bound target nucleic acid having a region at which a mutation is present to a plurality of probes; exposing the hybridized target nucleic acid and probe mixture to a degradation agent that preferentially degrades single-stranded nucleic acids; and detecting the presence of mutation in the target nucleic acid when a single-stranded region is degraded.

More specifically, the target nucleic acid (8) having a region with a mutation (22) is bound to a solid phase or semi-solid phase matrix (10). The target nucleic acid (8) is exposed to a plurality of probes (2) that are labeled by, for example, fluorescence. The target nucleic acid (8) and the plurality of probes (2) are incubated under optimal time, temperature, oligonucleotide concentration, salt type, and salt concentration conditions. Stringent hybridization conditions that maximize specific hybridization by improving bonding energy symmetry and providing similar melting temperature for each probe are employed. The hybridization conditions enable only complementary probes to hybridize to the target nucleic acid. Because no probe will be complementary to the region having a mutation (22), hybridization will not occur at that region, and the region will remain single-stranded.

After exposure to a degradation agent that preferentially degrades single-strand nucleic acid, the hybridization product is removed from solution by its bound end. The use of bound target nucleic acid enables a number of samples to be screened simultaneously by removing the bound portion from solution and then analyzing the solution phase for segments of hybridized (*i.e*., double-stranded) degradation product (26) indicative of the presence of a mutation in the target nucleic acid. The presence of one or more segments of hybridized degradation product (26) in solution is indicative that the target nucleic acid comprises a region having mutation (22) that was degraded by the degradation agent. The mutation is detected by exposing the solution to a light source in a spectrophotometer at the specific absorption wavelength, which reveals the appreciable quantities of fluorescing degradation product (26) indicative of a mutation (22).

Figure 2B illustrates an embodiment of the method of the invention wherein a mutation is not present in a target nucleic acid sample. In this embodiment, the target nucleic acid is bound to a solid phase or semi-solid phase matrix (10). The target nucleic acid is exposed to a plurality of probes (4) that are designed to hybridize to the target such that gaps separate one or more of the hybridized probes. The probe (4) designed to anneal to the area of the target nucleic acid most distal to the matrix (10) is labeled with a reporter (12), for example, a radionucleotide or a fluorophore.

The target nucleic acid and the plurality of probes (4) are incubated under optimal time, temperature, probe concentration, salt type, and salt concentration conditions. The plurality of probes (4) are complementary to different positions of the target nucleic acid such that hybridization of members of the plurality with a wild-type target results in a series of probes (4), along at least a portion of the target sequence, that are separated by a gap of single-stranded nucleic acid. Stringent hybridization conditions that maximize specific hybridization by improving bonding energy symmetry and providing similar melting temperatures for each probe are preferably employed. Those hybridization conditions enable only complementary probes to hybridize to the target nucleic acid.

The target nucleic acid and probe (4) hybridization product (18) is then exposed to an agent, or alternatively conditions employed with the agent, that do not cleave the target nucleic acid at the single-stranded gap separations, but that degrades a single stranded gap region where one or more probes failed to hybridize. The hybridization product (18) comprising the target nucleic acid and probes is then removed by its bound end from solution. The use of bound target nucleic acid enables a number of samples to be screened simultaneously by removing the bound portion from solution then analyzing the solution phase for degradation product indicative of a mutation. The absence of the reporter (12) remaining in solution indicates the absence of mutation in the target nucleic acid.

Figure 2C shows a flow chart diagram that illustrates an embodiment of the invention wherein a mutation is present in a target nucleic acid in the sample. According to this embodiment, the target nucleic acid (8) having a mutation (22) is bound to a solid phase or semi-solid phase matrix (10). The target nucleic acid (8) is exposed to a plurality of probes (4) designed to hybridize to the target (8) such that gaps separate one or more of the hybridized probes and the probe (4) designed to complement the area of the target nucleic acid (8) most distal to the matrix (10) is labeled with a reporter (12).

The target nucleic acid (8) and the plurality of probes (4) are incubated under the above-described stringent hybridization conditions. The conditions enable the plurality of probes (4) complementary to different positions of the target nucleic acid such that hybridization of members of the plurality with a wild-type target results in a series of probes (4), along at least a portion of the target sequence (8), that are separated by a gap of single-stranded nucleic acid. The stringent hybridization conditions enable only complementary probes to hybridize to the target nucleic acid.

The target nucleic acid (8) and probe (4) hybridization is then exposed to an agent, or alternatively conditions employed with the agent, that do not cleave the target nucleic acid at the single-stranded gap separations, but that degrade a single stranded gap region where one or more probes failed to hybridize. The hybridization product comprising the target nucleic acid and probes is then removed by its bound end from solution. The presence of one or more segments of hybridized degradation product (28) including the labeled reporter (12) remaining in solution indicates the presence of mutation (22) in the target nucleic acid (8), because the single stranded region of mutation (22) was degraded by the degradation agent.

The following example illustrates methods of the invention useful to detect a mutation in the mutation cluster region of the APC in samples prepared from stool.

### Example 1: Mutation detection in the APC Mutation Cluster Region

Methods of the invention are used to detect the C → T point mutation at codon 1450 in the APC mutation cluster region, *at* http://perso.curie.fr/Thierry.Soussi/APC.html (last visited Feb. 20, 2001). Any biological sample that comprises APC may be used, including, for example, a stool sample. For the analysis of stool samples, preferred methods of the invention comprise obtaining at least a cross-section or circumferential portion of a voided stool as taught in U.S. patent numbers 5,741,650, and 5,952,178, both of which are incorporated by reference herein. While a cross-sectional or circumferential portion of stool is desirable, methods provided herein are conducted on random samples obtained from voided stool, which include smears or scrapings. Once obtained, the stool specimen is homogenized. A preferable buffer for homogenization is one that contains at least 16 mM ethylenediaminetetraacetic acid (EDTA), as taught in copending, co-owned U.S. patent application serial number 09/491,093, incorporated by reference herein. It has been discovered that the use of at least 16 mM EDTA, and preferably 100 mM EDTA or greater improves the yield of nucleic acid from stool. Thus, a preferred buffer for stool homogenization comprises phosphate buffered saline, 20-100 mM NaCl or KCl, at least 16 mM EDTA, and optionally a detergent (such as SDS) and a proteinase (e.g., proteinase K).

After homogenization, nucleic acid is preferably isolated from the stool sample. Isolation or extraction of nucleic acid is not required in all methods of the invention, as methods of the invention can be adequately performed in homogenized stool without isolation of nucleic acids. In a preferred embodiment, however, homogenized stool is spun to create a supernatant containing nucleic acids, proteins, lipids, and other cellular debris. The supernatant is treated with a detergent and proteinase to degrade protein, and the nucleic acid is phenol-chloroform extracted. The extracted nucleic acids are then precipitated with alcohol. Other techniques can be used to isolate nucleic acid from the sample. Such techniques include hybrid capture, and amplification directly from the homogenized stool. Nucleic acids can be purified and/or isolated to the extent required by the screening assay to be employed.

The nucleic acid is then mixed with steptavidin coated Dynal beads, which provides a solid phase matrix. The nucleic acid and bead mixture is vortexed and incubated which binds the beads to the nucleic acid. The nucleic acid can be amplified by PCR, which requires the nucleic acid template to be mixed with binding and wash buffers. The nucleic acid mixture is vortexed. The supernatant is removed, and buffer is added. These steps are then repeated a number of times.

Nucleic acid probes designed to complement consecutive regions of the known APC mutation cluster region are employed. The probes are uniform in length and are fluorescently labeled. The probe and the target nucleic acid comprising a point mutation in codon 1450 are incubated under conditions that maximize hybridization selectivity. Probe melting temperature disparities are eliminated, improving selectivity, when a suitable combination of hybridization temperature, time, probe concentration, salt type and salt concentration conditions are employed. TMAC is the agent selected to improve hybridization selectivity.

The probes are designed to detect mutations at codon 1450 in the APC mutation cluster region. When hybridizing under these selective hybridization conditions, the presence of a single mutation in the mutation cluster region will prevent the complementary probe from hybridizing, such that a portion of the region remains single stranded.

Consecutive complementary probes are designed to hybridize to the wild type APC mutation cluster region where the 5' end of that region is (5'-CTCCACCACCTCCTCAA ACAGCTCAAACCAAGCG AGAAGTACCTAAAAATA -3', SEQ ID NO:1).

In the experiment, each probe comprises 17 nucleotides, and the 5' end of the complementary probe designed for the region of codon 1450 is (5'- CGCTTGGTTTGAGCTGT -3', SEQ ID NO: 2). The complimentary probe upstream of the codon 1450 point mutation region is (5'-TTGAGGAGGTGGTGGAG -3', SEQ ID NO: 3). The complimentary probe downstream of the 1450 point mutation region is (5'- TATTTTTAGGTACTTCT - 3', SEQ ID NO: 4). The probes and the target nucleic acid sample comprising the point mutation at codon 1450 in the mutation cluster region are incubated under conditions that maximize hybridization selectivity. The probe complimentary to the wild type region, SEQ ID No. 2, will not hybridize to the sequence comprising the point mutation at codon 1450 (C → T at the codon 1450 point mutation), (5'-ACAGCTCAAACCAAGTG -3', SEQ ID NO:5). The point mutation at codon 1450 prevents hybridization and the portion of the APC region containing the mutation will remain single stranded.

After hybridization, unhybridized probes are removed by washing the nucleic acid mixture under time, temperature, salt type and salt concentration conditions that preserve the nucleic acid:probe hybrids. Exposure to the enzyme S1 cleaves the target nucleic acid at the single-stranded region comprising the point mutation at codon 1450, where the complimentary probe failed to hybridize.

The degradation products are separated by gel electrophoresis and analyzed using a spectrophotometer. The presence of mutation is detected by the presence of one or more degradation products, each comprising double-stranded nucleic acids which fluoresce upon excitation at the appropriate spectrophotometer wavelength.

### Example 2: Probes Blocking Polymorphic Variants at Codon 1493

Generally, a single nucleotide polymorphism is associated with each region having 1,000 nucleotide base pairs. According to methods of the invention, associated polymorphic variants are factored into probe design such that associated polymorphisms are "blocked" and other alterations may be detected.

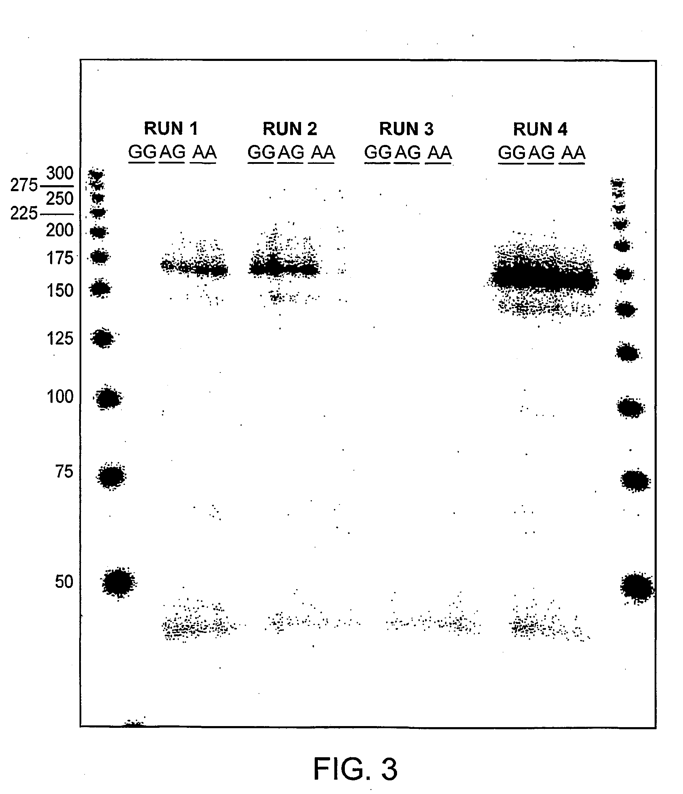

It is possible to avoid detecting a polymorphic variant as a false positive on a target nucleic acid. This provides an opportunity for de novo alteration detection on such targets. Figure 3 illustrates methods of the invention employed to block the G → A polymorphism at codon 1493 of the APC mutation cluster region, to enable de novo detection.

Any biological sample that comprises an associated polymorphism may be used, for example, a tissue, stool or blood sample. For this analysis, blood samples from six individuals were separately tested. The sample set included a polymorphic variant base at codon 1493. For the purposes of a control in this experiment, genotype sequencing was performed on the six individual samples prior to performing the analysis illustrated in Figure 3. The control sequence confirmed that the sample set being tested included two homozygous individuals having two G bases, two homozygous individuals having an A base and a G base, and two homozygous individuals having two A bases.

Each individual sample was analyzed as follows. Nucleic acid was extracted from each individual blood sample. The nucleic acid was then mixed with magnetic streptavidin coated Dynal beads, which provided a solid phase matrix. The nucleic acid and bead mixture was vortexed and incubated to bind the beads to the nucleic acid. The nucleic acid was thereafter amplified by PCR, which required the nucleic acid template to be mixed with binding and wash buffers. During the PCR process, the reverse PCR primer was biotinylated. The PCR product was denatured and made single stranded. The nucleic acid mixture was vortexed. The supernatant was removed, and buffer was added.

Nucleic acid probes were designed to compliment the target nucleic acid including the polymorphic variants in codon 1493 of the APC mutation cluster region. The probes are uniform in length are 17 base pairs long. The probes are designed so that after hybridization the probe positioned second in from the free end of the target nucleic acid (i.e., the end of the target nucleic acid that is not bound to the bead) has a P32 reporter on its 5' end. The probes and the target nucleic acid are hybridized at 59°C for one hour. The hybridization solution was a mixture of 3 molar tetramethyl ammonium chloride (TMA); 1mM EDTA; 10 mM phosphate buffer at a pH of 6.8; 5X of Denhardts solution; 0.04 mg/ml of yeast RNA; SDS at 0.1% of the mixture and between about 0.04 micromolar and about 0.64 micromolar of the probe mixture. The tube is exposed to a magnet that attracts the magnetic beads and retains the target nucleic acid that is bound to the beads and the supernatant is removed.

After hybridization, the unhybridized probes were removed by washing the nucleic acid mixture under conditions that preserve the nucleic acid:probe hybrids. The hybridization solution was washed a series of times under varying time and temperature conditions. The wash solution contained a mixture of 3 molar tetramethyl amonium chloride (TMA); 1mM EDTA; 10 mM phosphate buffer at a pH of 6.8; and SDS at 0.1 % of the mixture. After each wash, the tube is exposed to a magnet that attracts the magnetic beads and retains the target nucleic acid that is bound to the beads and the supernatant is removed. In the first wash, the hybridization solution was mixed with the wash solution at 59°C for 15 minutes. Thereafter, in the second wash, the hybridization solution was mixed with the wash solution at room temperature, about 22°C, for 1 minute. In the third wash, the hybridization solution was mixed with a the wash solution at room temperature, about 22°C, for about 1 minute.

In a test tube, the target nucleic acid was exposed to 0.015 unit per microliter of the enzyme S1, in the buffer containing sodium acetate at 50 mM, NaCl at 280 mM, and ZnSO₄ at 4.5 mM, per microliter of the cutting reaction for thirty minutes at room temperature, about 22°C. The reaction is a 100 microliter reaction. Exposure to the enzyme S1 cleaves any single stranded regions of the target nucleic acid. The tube is exposed to a magnet to attract the magnetic beads and retain the target nucleic acid that is bound to the beads. The supernatant is pipetted out of the tube and mixed with a load dye.

The supernatant is run on a 6% non-denaturing acrylamide gel at a rate of 1,200 Volt hours. The gel is exposed to an instant imager, which picks up radioactivity. The gel is analyzed for fragment size and any cuts in the target nucleic acid can be determined by the size of the product on the gel.

Referring to Figure 3, Run 1 shows experimental results where the six samples being tested were exposed to probes designed to complement the polymorphic variant where codon 1493 is a G. The six samples include two individuals having two G bases, two individuals having an A base and a G base, and two individuals having two A bases. Referring to the sample from the two individuals having two G bases, the product on the gel shows that exposure to the agent, S l, did not cut the target. The target nucleic acid was not cut because the probes designed to complement the polymorphic variant where codon 1493 is a G hybridized to the target nucleic acid of the two individuals having two G bases. Referring again to Run 1, samples from two individuals having an A base and a G base were exposed to the probes designed to complement the polymorphic variant where codon 1493 is a G. The probes hybridized to the G variant of the two individuals having an A base and a G base. However, no probe hybridized to the polymorphic variant of the target sample where codon 1493 is an A and upon exposure to the agent S1, the single stranded region at codon 1493 was cut generating product on the gel. Finally, in Run 1, samples from two individuals having two A bases were exposed to the probes designed to complement the polymorphic variant where codon 1493 is a G. No probe hybridized to the polymorphic variant of the two individuals having two A bases, and exposure to the agent, S1, cut the target at the region of the polymorphic variant generating product on the gel.

Referring to Figure 3, Run 2 shows experimental results where the six samples being tested were exposed to probes designed to complement the polymorphic variant where codon 1493 is an A. Again, the six samples include two individuals having two G bases, two individuals having an A base and a G base, and two individuals having two A bases. Referring to the sample from the two individuals having two G bases, the product on the gel shows exposure to the agent, S1, cut the target at the region of the polymorphic variant leaving product on the gel. The target nucleic acid was cut because the probes including the polymorphic variant where codon 1493 is A failed to hybridize to the target nucleic acid of the two individuals having two G bases. Referring again to Run 2, samples from two individuals having an A base and a G base were exposed to the probes designed to complement the polymorphic variant where codon 1493 is an A. The probes hybridized to the A variant of the two individuals having an A base and a G base. However, no probe hybridized to the polymorphic variant of the target sample where codon 1493 is G and upon exposure to the agent S1, the single stranded region at codon 1493 was cut generating product on the gel. Finally, in Run 2 samples from two individuals having two A bases were exposed to the probes designed to complement the polymorphic variant where codon 1493 is a A. The product on the gel shows exposure to the agent, S1, did not cut the target. The target nucleic acid was not cut because the probes designed to complement the polymorphic variant where codon 1493 is an A hybridized to the target nucleic acid of the two individuals having two A bases.

Referring to Figure 3, Run 3 shows experimental results where the six samples being tested were exposed to two different sets of probes: one designed to complement the polymorphic variant where codon 1493 is an A and one designed to complement the polymorphic variant where codon 1493 is a G. Again, the six samples include two individuals having two G bases, two individuals having an A base and a G base, and two individuals having two A bases. Referring again to Run 3, exposure to sets of probes designed to complement both the polymorphic variant A and the polymorphic variant G succeeds in blocking the codon 1493 region in all six samples. Thus, both polymophic variant probes successfully block the polymorphic variants on the target nucleic acids tested and, according to methods of the invention, any other alterations on the target may be detected.

Finally, referring to Figure 3, Run 4 shows experimental results where, again, six samples were tested, the samples from two individuals having two G bases, two individuals having an A base and a G base, and two individuals having two A bases. The six samples being tested were exposed to probes where no probe was designed to complement the polymorphic variants present in the six samples. Referring again to Run 4, upon exposure to the agent S1, the single stranded region at codon 1493 for both the polymorphic variant where codon 1493 is an A and the polymorphic variant where codon 1493 is a G was cut generating product on the gel. Thus, according to methods of the invention, probes may be designed to block the G → A polymorphism at codon 1493 of the APC to enable detection of other alterations in the target nucleic acid.

### Example 3: Mutation detection by probe reporting moiety signal

This example does not illustrate the claimed invention.

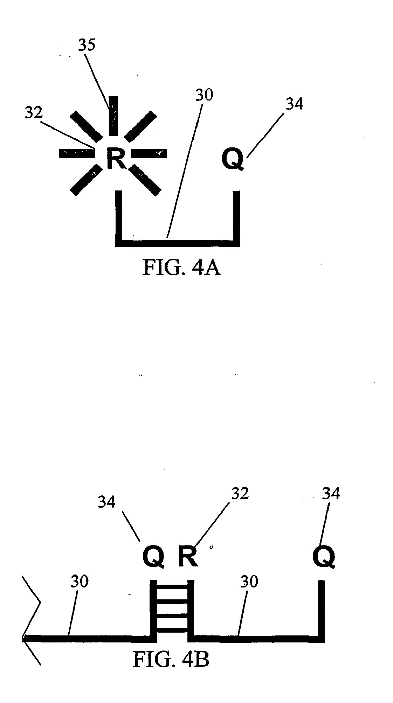

In another embodiment, referring to Figure 4A, a quenching moiety (34) is placed at one end and a reporting moiety (32) is placed at the other end of each of the probes (30) such that the absence of hybridization of one probe results in the reporter of an adjacent probe failing to be quenched resulting in a reporter signal (35). Accordingly, an alteration is detected by the reporting moiety (32) signal (35) that results when a probe (30) fails to hybridize to the target at the site of the alteration. The reporting moiety (32) or reporter may be detected via a fluorescent plate reader or alternatively with a gel apparatus equipped with fluorescent detection. A degradation agent is not required to detect the presence or absence of mutation in the target nucleic acid in this method of the invention. Accordingly, serial analysis may be avoided by employing this method of the invention.

Figure 4B illustrates the reporting moiety (32) of a first probe (30) annealed to the quenching moiety (34) of a second probe (30). The quenching moiety (34) quenches or suppresses the reporting moiety (32) signal (35). The quenching moiety (34) is conjugated to an arm on one end of probe (30) and the reporting moiety (32) is conjugated to an arm on the other end of probe (30), and each arm may include a sequence. In one embodiment, the sequences on the arm conjugated to the quenching moiety (34) are designed to anneal to the arm conjugated to any reporting moiety (32). In one embodiment, the sequences on the arms are not specific to the sequence complementary to the target nucleic acid. The sequences on each arm may range between about 1 base pair and about 7 base pairs.

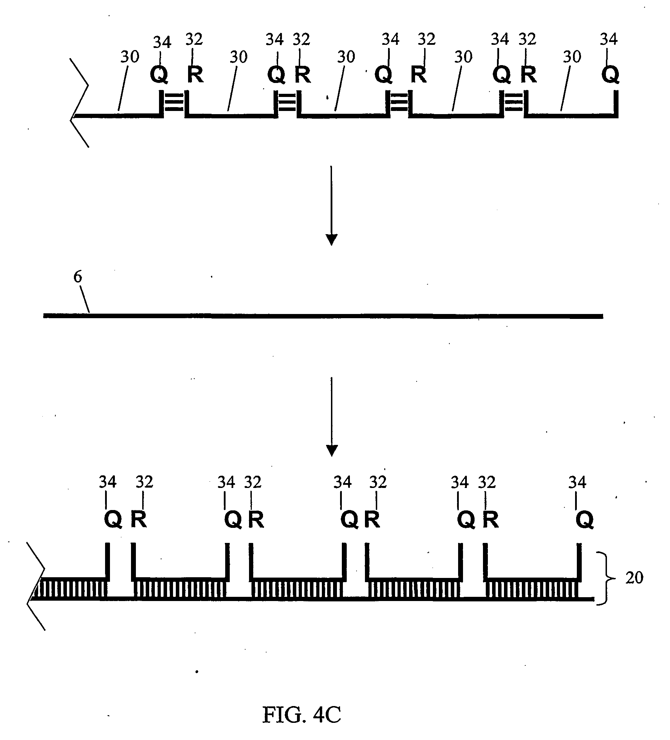

Figure 4C shows a flow chart diagram that illustrates a method of the invention using probes having a reporting moiety (32) and a quenching moiety (34) and wherein no mutation is present in a target nucleic acid. According to this method, when a series of probes (30), as illustrated in Figures 4A and 4B, are mixed together, for example in solution, the arm conjugated to each quenching moiety (34) will anneal to the arm conjugated to each reporting moiety (32). Accordingly, each reporting moiety (32) is quenched by each adjacent quenching moiety (34) and the probe (30) mixture in solution presents no reporting signal.

Thereafter, a target nucleic acid (6) is exposed to the probe (30) mixture to form a hybridization product (20). According to the method, the hybridization conditions enable only complementary probes to hybridize to the target nucleic acid (6). When the hybridization product (20) fails to present any signal, the absence of mutation in the target nucleic acid (6) is detected.

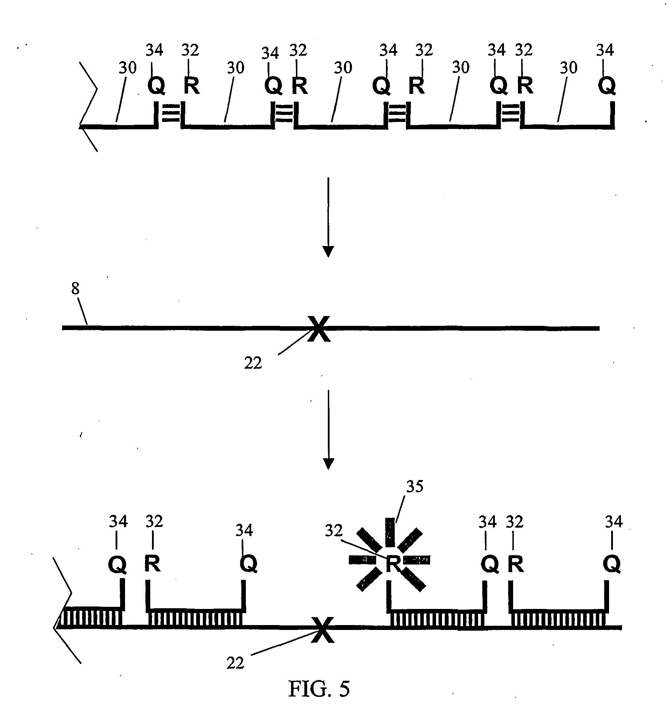

Figure 5 shows a flow chart diagram that illustrates a method of the invention using probes having a reporting moiety (32) and a quenching moiety (34) and wherein a mutation (22) is present in a target nucleic acid (8). According to this method, when a mixture of probes (30), as described above with reference to Figures 4A-4C, is prepared, the probe (30) mixture presents no reporting signal (35).

Thereafter, a target nucleic acid (8) is exposed to the probe (30) mixture to form a hybridization product (20). The hybridization conditions enable only complementary probes (30) to hybridize to the target nucleic acid (8). Because no probe (30) will be complementary to the region having a mutation (22), hybridization will not occur in the mutation (22) region and the mutation region will remain single-stranded. When a probe (30) fails to hybridize in the region of the mutation (22) then the reporting moiety (32) adjacent the region having the mutation (22) will present a signal (35), the signal (35) indicating the presence of mutation (22).

In the examples described above, embodiments of the invention wherein a target nucleic acid contains no mutation are described separately from embodiments of the invention wherein a target nucleic acid contains a mutation. However, according to the invention, a sample may be heterogeneous and contain target nucleic acid molecules that are mutant or altered in addition to target nucleic acid molecules that are non-mutant or non-altered.

Methods of the invention are useful to detect a mutant or altered target nucleic acid in a heterogeneous mixture containing non-mutant or non-altered target nucleic acid, because the non-mutant or non-altered nucleic acid does not interfere with the generation of a signal due to the presence of the mutant or altered nucleic acid. In preferred embodiments, a detection assay of the invention is performed on a sample or sample fraction that has been enriched for mutant or altered target nucleic acid using methods described herein or known in the art.

## Claims

1. A method for detecting an alteration in a target nucleic acid suspected to be in a biological sample, the method comprising the steps of:
a) adding, to a biological sample suspected to contain a target nucleic acid, a plurality of single-stranded peptide nucleic acids that hybridize contiguously to a region of said target nucleic acid if said region is unaltered;
b) adding to said biological sample an agent that degrades single-stranded nucleic acids; and,
c) detecting an alteration in said target nucleic acid as the presence of a degradation product from steps a) and b) resulting from degradation within said region of said target nucleic acid.

2. The method of claim 1, wherein at least one member of said plurality of single-stranded peptide nucleic acids comprises an end with a donor and an end with a quencher.

3. The method of claim 1, further comprising the steps of:
a) diluting a biological sample; and,
b) separating said biological sample into separate sample fractions.

4. A method for detecting an alteration in a target nucleic acid suspected to be in a biological sample, the method comprising the steps of:
a) adding, to a biological sample suspected to contain a target nucleic acid, a plurality of probes that hybridize to a region of said target nucleic acid such that a gap separates one or more of the adjacent hybridized probes, if said region is unaltered;
b) adding to said biological sample an agent under conditions that degrade single-stranded nucleic acids larger than said gap size; and
c) detecting an alteration in said target nucleic acid as the presence of a degradation product from steps a) and b) resulting from degradation within said single-stranded region of said target nucleic acid.

5. A method for detecting an alteration in a polymorphic target nucleic acid suspected to be in a biological sample, the method comprising the steps of:
a) adding, to a biological sample suspected to contain a polymorphic target nucleic acid, a plurality of probes, said plurality comprising probes complementary to each polymorphic variant of said target nucleic acid, such that said probes hybridize contiguously to a region of said target nucleic acid if said region is unaltered;
b) adding to said biological sample an agent that degrades single-stranded nucleic acids; and,
c) detecting an alteration in said target nucleic acid as the presence of a degradation product from steps a) and b) resulting from degradation within said region of said target nucleic acid.

6. A method for detecting an alteration in a target nucleic acid suspected to be in a biological sample, the method comprising the steps of:
a) adding, to a biological sample suspected to contain a target nucleic acid, a plurality of single-stranded nucleic acids that hybridize contiguously to a region of said target nucleic acid if said region is unaltered;
b) adding to said biological sample an agent that degrades single-stranded nucleic acids; and,
c) detecting an alteration in said target nucleic acid as the presence of a degradation product from steps a) and b) resulting from degradation within said region of said target nucleic acid.

7. The method of any of the preceding claims, wherein said alteration is a disease-associated mutation; and optionally: (a) wherein said disease is cancer; or (b) said method further comprising the step of determining the identity of said alteration in said target nucleic acid.

8. The method of claim 1 or claim 6, wherein: (a) at least one member of said plurality of single-stranded nucleic acids comprises a detectable label; or (b) wherein said target nucleic acid suspected of being in said biological sample comprises a detectable label; and in either case (a) or (b) optionally wherein said detectable label is selected from the group consisting of a fluorescent tag, a radioactive isotope, and a molecular weight marker.

9. The method of claim 1 or claim 6 wherein: (a) each member of said plurality of single-stranded nucleic acids is between about 8 and 30 nucleotides long; (b) each member of said plurality of single-stranded nucleic acids has an approximately equivalent hybridization melting temperature with said target nucleic acid; (c) said target nucleic acid is bound to a solid support; and optionally wherein the 5' end of said target nucleic acid is bound to said solid support, or the 3' end of said target nucleic acid is bound to said solid support; (d) said biological sample comprises a tissue or body fluid; (e) said agent is an enzyme, and optionally wherein said enzyme is selected from the group consisting of S1, MutY, MutS and mungbean nuclease; (f) said agent is a chemical agent; (g) said alteration is a disease-associated mutation and is selected from the group consisting of nucleotide insertions, deletions, rearrangements, transitions, translations, transversions, and substitutions; (h) said biological sample comprises a tissue or body fluid selected from the group consisting of sputum, pancreatic fluid, bile, lymph, plasma, urine, cerebrospinal fluid, seminal fluid, saliva, breast nipple aspirate, pus, biopsy tissue, fetal cells, and amniotic fluid; (i) said biological sample is a stool sample; (j) said alteration is a single nucleotide polymorphism; (k) said alteration is inherited; or (l) said alteration exists as a subpopulation in a heterogeneous sample.

10. A method according to any of the preceding claims, wherein the agent that degrades single-stranded nucleic acids selectively cleaves single-stranded nucleic acid.

## Patentansprüche

1. Verfahren zum Nachweis einer Mutation in einer Zielnukleinsäure, von der angenommen wird, dass sie in einer biologischen Probe enthalten ist, wobei das Verfahren die nachfolgenden Schritte umfasst:
a) Hinzufügen einer Vielzahl von einsträngigen Peptidnukleinsäuren, die sich benachbart einer Region der genannten Zielnukleinsäure hybridisieren, falls die genannte Region nicht mutiert ist, zu einer biologischen Probe, von der angenommen wird, dass diese eine Zielnukleinsäure enthält;
b) Hinzufügen eines Mittels zur genannten biologischen Probe, das einsträngige Nukleinsäuren zersetzt; und
c) Nachweisen einer Mutation in der genannten Zielnukleinsäure durch die Anwesenheit eines Zersetzungsproduktes aus den Schritten a) und b), das durch die Zersetzung innerhalb der genannten Region der genannten Zielnukleinsäure entsteht.

2. Verfahren nach Anspruch 1, wobei wenigstens ein Mitglied der genannten Vielzahl von einsträngigen Peptidnukleinsäuren ein Ende mit einem Spender und ein Ende mit einem Abschreckmittel umfasst.

3. Verfahren nach Anspruch 1, ferner umfassend die nachfolgenden Schritte:
a) Verdünnen einer biologischen Probe; und
b) Trennen der genannten biologischen Probe in einzelne Probenteile.

4. Verfahren zum Nachweis einer Mutation in einer Zielnukleinsäure, von der angenommen wird, dass diese in einer biologischen Probe enthalten ist, wobei das Verfahren die nachfolgenden Schritte umfasst:
a) Hinzufügen einer Vielzahl von Sonden zu einer biologischen Probe, von der angenommen wird, dass diese eine Zielnukleinsäure enthält, die sich derart an eine Region der genannten Zielnukleinsäure hybridisieren, dass ein Spalt eine oder mehrere der benachbarten, hybridisierten Sonden trennt, falls die genannte Region nicht mutiert ist;
b) Hinzufügen eines Mittels zu einer biologischen Probe unter Bedingungen, welche die einsträngigen Nukleinsäuren zersetzten, die größer als die genannte Spaltgröße sind; und
c) Nachweisen einer Mutation in der genannten Zielnukleinsäure durch die Anwesenheit eines Zersetzungsproduktes aus den Schritten a) und b), das durch die Zersetzung innerhalb der genannten einsträngigen Region der genannten Zielnukleinsäure entsteht.

5. Verfahren zum Nachweis einer Mutation in einer polymorphen Zielnukleinsäure, von der angenommen wird, dass diese in einer biologischen Probe enthalten ist, wobei das Verfahren die nachfolgenden Schritte umfasst:
a) Hinzufügen einer Vielzahl von Sonden zu einer biologischen Probe, von der angenommen wird, dass diese eine polymorphe Zielnukleinsäure enthält, wobei die Vielzahl Sonden enthält, die auf jede der polymorphen Varianten der genannten Zielnukleinsäure derart abgestimmt ist, dass sich die genannten Sonden benachbart zu einer Region der genannten Zielnukleinsäure hybridisieren, falls die genannte Region nicht mutiert ist;
b) Hinzufügen eines Mittels zur genannten biologischen Probe, das die einsträngigen Nukleinsäuren zersetzt; und
c) Nachweisen einer Mutation in der genannten Zielnukleinsäure durch die Anwesenheit eines Zersetzungsproduktes aus den Schritten a) und b), das durch die Zersetzung innerhalb der genannten Region der genannten Zielnukleinsäure entsteht.

6. Verfahren zum Nachweis einer Mutation in einer Zielnukleinsäure, von der angenommen wird, dass diese in einer biologischen Probe enthalten ist, wobei das Verfahren die nachfolgenden Schritte umfasst:
a) Hinzufügen einer Vielzahl von einsträngigen Nukleinsäuren, welche sich benachbart zu einer Region der genannten Zielnukleinsäure hybridisieren, falls die genannte Region nicht mutiert ist, zu einer biologischen Probe, von der angenommen wird, dass diese eine Zielnukleinsäure enthält;
b) Hinzufügen eines Mittels zur genannten biologischen Probe, welches einsträngige Nukleinsäuren zersetzt; und
c) Nachweisen einer Mutation in der genannten Zielnukleinsäure durch die Anwesenheit eines Zersetzungsprodukts aus den Schritten a) und b), das durch die Zersetzung innerhalb der genannten Region der genannten Zielnukleinsäure entsteht.

7. Verfahren nach einem der vorstehenden Ansprüche, wobei die genannte Mutation eine krankheitsassoziierte Mutation ist; und wahlweise: (a) wobei es sich bei der genannten Krankheit um Krebs handelt oder (b) wobei das genannte Verfahren ferner den Schritt des Feststellens der Identität der genannten Mutation in der genannten Zielnukleinsäure umfasst.

8. Verfahren nach Anspruch 1 oder Anspruch 6, wobei: (a) wenigstens ein Mitglied der genannten Vielzahl von einsträngigen Nukleinsäuren eine nachweisbare Markierung umfasst; oder (b) wobei die genannte Zielnukleinsäure, von der angenommen wird, dass diese in einer biologischen Probe enthalten ist, eine nachweisbare Markierung umfasst; und wahlweise in einem der Fälle (a) oder (b) wobei die nachweisbare Markierung ausgewählt ist aus der Gruppe bestehend aus einer Fluoreszenzmarkierung, einem radioaktiven Isotop sowie eines molekularen Gewichtsmarkers.

9. Verfahren nach Anspruch 1 oder Anspruch 6, wobei: (a) jedes Mitglied der genannten Vielzahl von einsträngigen Nukleinsäuren zwischen etwa 8 und 30 Nukleotide lang ist; (b) jedes Mitglied der genannten Vielzahl von einsträngigen Nukleinsäuren eine ungefähre, äquivalente Hybridisierungs-Schmelztemperatur mit der genannten Zielnukleinsäure aufweist; (c) die genannte Zielnukleinsäure an eine fest Stütze gebunden ist; und wahlweise wobei das 5'-Ende der genannten Zielnukleinsäure an die genannte feste Stütze gebunden ist oder das 3'-Ende der genannten Zielnukleinsäure an die genante feste Stütze gebunden ist; (d) die genannte biologische Probe ein Gewebe oder eine Körperflüssigkeit umfasst; (e) das genannte Mittel ein Enzym ist und wahlweise wobei das genannte Enzym ausgewählt ist aus der Gruppe bestehend aus S1, MutY, MutS sowie der Mung Bean-Nuklease; (f) das genannte Mittel ein chemisches Mittel ist; (g) die genannte Mutation eine krankheitsassoziierte Mutation und ausgewählt ist aus der Gruppe bestehend aus Nukleotidinsertionen, Deletionen, Transitionen, Translationen, Transversionen und Substitutionen; (h) die genannte biologische Probe ein Gewebe oder eine Körperflüssigkeit umfasst, die ausgewählt sind aus der Gruppe bestehend aus Sputum, Bauchspeicheldrüsen-Flüssigkeit, Gallenflüssigkeit, Lymphknoten-, Plasma-, Urin-, Zerebrospinalflüssigkeit, Samenflüssigkeit, Speichelflüssigkeit, Brustwarzenaspirata, Eiter, Biopsiegewebe, Fötalzellen sowie Fruchtwasser; (i) die genannte biologische Probe eine Stuhlprobe ist; (j) die genannte Mutation eine einzelne Nukleotid-Polymorphie ist; (k) die genannte Mutation vererblich ist; oder (l) die genannte Mutation als eine Teilgesamtheit in einer heterogenen Probe existiert.

10. Verfahren nach einem der vorstehenden Ansprüche, wobei das die einsträngigen Nukleinsäuren zersetzende Mittel wahlweise einsträngige Nukleinsäuren spaltet.

## Revendications

1. Procédé de détection d'une altération au niveau d'un acide nucléique cible dont la présence est suspectée dans un échantillon biologique, le procédé comprenant les étapes consistant à :
a) ajouter, à un échantillon biologique suspecté de contenir un acide nucléique cible, une pluralité d'acides nucléiques peptidiques simple brin qui s'hybrident de façon contiguë à une région dudit acide nucléique cible si ladite région n'est pas altérée ;
b) à ajouter audit échantillon biologique un agent qui dégrade les acides nucléique simple brin ; et
c) à détecter une altération au niveau dudit acide nucléique cible sous forme de la présence d'un produit de dégradation des étapes a) et b) provenant de la dégradation à l'intérieur de ladite région dudit acide nucléique cible.

2. Procédé selon la revendication 1, dans lequel au moins un membre de ladite pluralité d'acides nucléiques peptidiques simple brin comprend une extrémité avec un donneur et une extrémité avec un désactivateur.

3. Procédé selon la revendication 1, comprenant également les étapes consistant à :
a) diluer l'échantillon biologique ; et
b) séparer ledit échantillon biologique en plusieurs fractions d'échantillons.

4. Procédé de détection d'une altération au niveau d'un acide nucléique cible dont la présence est suspectée dans un échantillon biologique, le procédé comprenant les étapes consistant à :
a) ajouter, à un échantillon biologique suspecté de contenir un acide nucléique cible, une pluralité de sondes qui s'hybride à une région dudit acide nucléique cible de sorte qu'un espace sépare une ou plusieurs des sondes hybridées adjacentes, si la région n'est pas altérée ;
b) ajouter audit échantillon biologique un agent dans des conditions qui dégradent les acides nucléique simple brin ayant une taille supérieure à la taille dudit espace ; et
c) détecter une altération au niveau dudit acide nucléique cible sous forme de la présence d'un produit de dégradation des étapes a) et b) provenant de la dégradation à l'intérieur de la région simple brin dudit acide nucléique cible.

5. Procédé de détection d'une altération au niveau d'un acide nucléique cible polymorphe dont la présence est suspectée dans un échantillon biologique, le procédé comprenant les étapes consistant à :
a) ajouter, à un échantillon biologique suspecté de contenir un acide nucléique cible polymorphe, une pluralité de sondes, ladite pluralité comprenant des sondes complémentaires à chaque variant polymorphique dudit acide nucléique cible, de sorte que lesdites sondes s'hybrident de façon contiguë à une région dudit acide nucléique cible si la région n'est pas altérée ;
b) ajouter audit échantillon biologique un agent qui dégrade les acides nucléiques simple brin ; et
c) détecter une altération au niveau dudit acide nucléique sous forme de la présence d'un produit de dégradation des étapes a) et b) provenant de la dégradation à l'intérieur de ladite région dudit acide nucléique cible.

6. Procédé de détection d'une altération au niveau d'un acide nucléique cible dont la présence est suspectée dans un échantillon biologique, le procédé comprenant les étapes consistant à :
a) ajouter, à un échantillon biologique suspecté de contenir un acide nucléique cible, une pluralité d'acides nucléiques simple brin, qui s'hybrident de façon contiguë à une région dudit acide nucléique cible si ladite région n'est pas altérée ;
b) ajouter audit échantillon biologique un agent qui dégrade les acides nucléiques simple brins ; et
c) détecter une altération au niveau dudit acide nucléique sous forme de la présence d'un produit de dégradation des étapes a) et b) provenant de la dégradation à l'intérieur de ladite région dudit acide nucléique cible.

7. Procédé selon l'une quelconque des revendications précédentes, dans lequel ladite altération est une mutation associée à une maladie ; et éventuellement : (a) dans lequel ladite maladie est le cancer ; ou (b) ledit procédé comprenant également l'étape consistant à déterminer l'identité de ladite altération dans ledit acide nucléique cible.

8. Procédé selon la revendication 1 ou de la revendication 6, dans lequel : (a) au moins un membre de ladite pluralité d'acides nucléiques simple brin comprend une étiquette détectable ; ou (b) dans lequel ledit acide nucléique cible dont la présence est suspectée dans ledit échantillon biologique comprend une étiquette détectable ; et dans chaque cas (a) ou (b) éventuellement dans lequel ladite étiquette détectable est choisi à partir d'un groupe consistant d'un marqueur fluorescent, un isotope radioactif et un marqueur du poids moléculaire.

9. Procédé selon la revendication 1 ou la revendication 6 dans lequel : (a) chaque membre de la pluralité d'acides nucléiquessimple brin à une longueur d'environ 8 à 30 nucléotides ; (b) chaque membre de la pluralité d'acides nucléiques simple brin a une température de fusion d'hybridation approximativement équivalente à celle dudit acide nucléique cible ; (c) ledit acide nucléique cible est fixé à un support solide, ou l'extrémité 3' dudit acide nucléique cible est lié à un support solide ; (d) ledit échantillon biologique comprend un tissu ou un fluide corporel ; (e) ledit agent est une enzyme, et éventuellement ladite enzyme est choisie à partir d'un groupe consistant de SI, MutY, MutS et la nucléase du haricot mungo ; (f) ledit agent est un agent chimique ; (g) ladite altération est une mutation associée à une maladie et elle est choisie à partir d'un groupe consistant en insertions, délétions, réarrangements, transitions, traductions, transversions et substitutions de nucléotides; (h) ledit échantillon biologique comprend un tissu ou un fluide corporel sélectionné à partir du groupe consistant de crachat, fluide pancréatique, bile, lymphe, plasma, urine, liquide cérébrospinal, liquide séminale, salive, aspirat de téton des seins, pus, biopsie de tissu, cellules foetales et liquide amniotique ; (i) ledit échantillon biologique est un échantillon de selles ; (j) ladite altération est un polymorphisme d'un seul nucléotide ; (k) ladite altération est héréditaire ou (1) ladite altération existe sous forme d'une sous population dans un échantillon hétérogène.

10. Procédé selon l'une quelconque des revendications précédentes, dans lequel l'agent qui dégrade les acides nucléiquessimple brin clivent sélectivement les acides nucléiques simple brin.
